# FASCICULE DE BREVET EUROPEEN

(11) **EP 0 648 488 B1**
(45) Date de publication et mention de la délivrance du brevet: **29.11.2000**
(21) Numéro de dépôt: 94402055.1
(22) Date de dépôt: 14.09.1994
(51) Int. Cl.: A61K 31/00, A61K 31/05, A61K 31/495, A61K 31/095, A61K 31/12, A61K 31/405, A61K 35/78, A61K 7/06

(54) **Procédé pour modifier la pousse des poils et/ou des cheveux et compositions utilisables à cet effet**
Verfahren zur Modifizierung des Wachstums von Körperhaaren und/oder Haaren und hierfür verwendbare Zusammensetzungen
Method to alter hairgrowth and compositions therefor

(30) Priorité: 13.10.1993 FR 9312178
(43) Date de publication de la demande: 19.04.1995
(73) Titulaire: L'OREAL, 75008 Paris (FR)
(72) Inventeur: Duranton, Albert, F-75018 Paris (FR); De Lacharriere, Olivier, F-75015 Paris (FR)
(74) Mandataire: Tezier Herman, Béatrice

(56) Documents cités:
- WO-A-92/00057
- WO-A-94/27563
- WO-A-94/27586
- FR-A- 2 380 775
- JP-A- 1 096 126
- CONTEMP. REV. OBSTET. GYNAECOL., vol.4, no.2, 1992 pages 90 - 101 Z.M. VAN DER SPUY 'Management of hyperandrogenism.'
- J. PHARM. SOC. JPN, vol.113, no.10, 1993 pages 718 - 724 N. KOBAYASHI ET AL. 'Effect of leaves of Ginko biloba on hair regrowth in C3H strain mice.'
- J. LIPID RES., vol.34, no.9, 1993 pages 1505 - 1514 A.N. BAER ET AL. 'Fatty acid oxygenase activity of human hair roots.'

## Description

La présente invention concerne, dans son aspect le plus général, un procédé de traitement pour modifier la pousse des poils et/ou des cheveux. Plus particulièrement, elle concerne un procédé permettant au choix, et ceci essentiellement selon la nature des produits qui sont utilisés pour sa mise en oeuvre, soit de favoriser la pousse et/ou limiter la chute desdits poils et/ou cheveux, soit, au contraire, de diminuer ou empêcher la pousse de ces derniers.

Elle concerne également divers types de compositions destinées notamment à la mise en oeuvre dudit procédé, ainsi que certaines de leurs utilisations spécifiques.

Dans l'art antérieur la demande de brevet FR-A-2380775 décrit une composition favorisant un ralentissement de la pousse des poils comprenant une ou plusieurs lipoxygénase associée à un substrat de l'enzyme.
La publication dans "Journal of the Pharmaceutical Society of Japan", vol.113, n°10, 1993, page 718-724 évoque l'effet d'un extrait de Gingko biloba sur la pousse des cheveux.
La demande WO-92-00057 décrit le traitement de l'alopécie par l'utilisation du kétoconazole.
La demande WO-94-27563 décrit la réduction de la pousse des cheveux par application d'un inhibiteur de 5-lipoxygénase.
La demande WO-94-27586 décrit la réduction de la pousse des cheveux par application d'un inhibiteur de 5-cyclooxygénase.
La demande JP-A1096126 décrit l'utilisation des flavonoïdes pour traiter l'alopécie et/ou l'hirsutisme.

On sait que certains acides gras polyinsaturés, en particulier ceux présentant 20 atomes de carbone, tels que l'acide arachidonique, l'acide dihomo-γ-linolénique ou bien encore l'acide éicosapentaénoïque, peuvent être transformés *in vivo*, sous l'action de certaines enzymes spécifiques contenues dans les cellules vivantes, en particulier les cellules épithéliales, en certains autres composés de type éicosanoïdes utiles à l'organisme.

Ainsi, il est connu que les enzymes dites cyclo-oxygénases génèrent, à partir des différents acides gras mentionnés ci-dessus, des éicosanoïdes de type prostaglandines et thromboxanes, et que les enzymes dites lipoxygénases sont, quant à elles, responsables de la formation des éicosanoïdes de type leukotriènes et autres acides acycliques hydroxylés à 20 atomes de carbone. Un même acide gras polyinsaturé donné (ou substrat) pourra donner lieu, selon la nature de l'enzyme avec laquelle il aura réagi en premier, à la formation de plusieurs métabolites différents, à savoir par exemple des prostaglandines et des leukotriènes.

Les acides gras polyinsaturés notamment en C₂₀ (matières premières réactives) appelés à être métabolisés sous l'action spécifique des enzymes cyclo-oxygénases et lipoxygénases sont généralement apportés à l'organisme par l'intermédiaire de certains aliments, en particulier de certaines huiles naturelles d'origine animale ou végétale, cet apport pouvant alors se faire soit sous une forme directe (c'est le cas par exemple de l'acide arachidonique, qui est présent tel quel dans les blancs d'oeufs), soit indirectement sous la forme de composés précurseurs (composés que l'on appelle aussi "acides gras essentiels", qui sont eux-mêmes des acides gras insaturés, généralement en C₁₈-C₂₂, tels que les acides linoléïque, α-linolénique et β-linolénique) qui seront transformés par le métabolisme humain, selon des mécanismes complexes qu'il n'est pas nécessaire de détailler ici, en substrats convenables (c'est à dire métabolisables) pour cyclo-oxygénases et lipoxygénases.

Or, après d'importantes recherches, il a maintenant été trouvé par la Demanderesse que les transformations enzymatiques ci-dessus exposées, et les différents produits de réaction qui en résultent, ont une influence non négligeable sur les mécanismes de pousse du poil et/ou du cheveu, et qu'en privilégiant (selon des techniques exposées ci-après) l'une ou l'autre des deux voies enzymatiques cyclo-oxygénases ou lipoxygénases au sein des cellules de la peau, on pouvait, de façon tout à fait inattendue et surprenante, modifier de manière substantielle la pousse des poils et/ou des cheveux.

Plus précisément encore, il a été trouvé qu'en privilégiant la voie des cyclo-oxygénases on peut alors favoriser la pousse des poils et/ou des cheveux et/ou lutter contre leur chute, et qu'en privilégiant la voie des lipoxygénases, on peut au contraire ralentir et/ou empêcher la pousse de ces derniers.

Il a par ailleurs été trouvé que, d'un point de vue pratique, il est possible de privilégier une voie enzymatique donnée de plusieurs manières différentes, comme précisé ci-dessous. Toutes ces variantes reposent sur le même principe de base, à savoir apporter à l'organisme, en particulier aux cellules de la peau, des substances destinées soit à inhiber, soit au contraire à stimuler, l'action des enzymes cyclo-oxygénases ou lipoxygénases, le choix desdites substances se faisant bien entendu en fonction de l'effet technique recherché.

Ainsi, si l'on cherche à favoriser la pousse et/ou limiter la chute des poils et/ou des cheveux (ou inversement si l'on cherche à ralentir et/ou empêcher la pousse de ces derniers), et compte tenu du fait qu'il convient dans ce cas, comme indiqué précédemment, de privilégier la voie des cyclo-oxygénases (ou inversement de privilégier la voie des lipoxygénases), on peut alors procéder, au choix, selon l'une au moins des façons suivantes : soit on fait appel à un ou plusieurs inhibiteurs des lipoxygénases (ou inversement des cyclo-oxygénases), soit on fait appel à un ou plusieurs stimulateurs ou agonistes des cyclo-oxygénases (ou inversement des lipoxygénases), soit on fait appel à un ou plusieurs inhibiteurs des lipoxygénases (ou inversement des cyclo-oxygénases) en association avec un ou plusieurs stimulateurs ou agonistes des cyclo-oxygénases (ou inversement des lipoxygénases), soit encore on fait appel à une ou plusieurs substances présentant la propriété d'être à la fois et simultanément des inhibiteurs des lipoxygénases (ou inversement des cyclo-oxygénases) et des stimulateurs ou agonistes des cyclo-oxygénases (ou inversement des lipoxygénases).

En résumé, on peut donc privilégier une voie enzymatique donnée par stimulation directe de cette voie et/ou par inhibition de la voie "contraire". Les meilleurs résultats sont généralement obtenus en cumulant les deux effets.

Enfin, il a par ailleurs été trouvé que l'on obtenait des résultats particulièrement remarquables, tant dans un procédé de traitement pour favoriser la pousse des poils et/ou des cheveux que dans celui visant au contraire à limiter cette pousse, lorsque l'utilisation desdits inhibiteurs et stimulateurs ou agonistes des voies enzymatiques ci-dessus était en outre couplée avec l'utilisation d'au moins un substrat directement métabolisable par les lipoxygénases et les cyclo-oxygénases et/ou d'au moins un précurseur dudit substrat (effet de synergie).

Toutes ces découvertes sont à la base de la présente invention.

Dans l'exposé qui suit de la présente invention, on entend désigner :
- par "substrat" convenable pour lipoxygénases et cyclo-oxygénases, toute substance qui, telle quelle, peut être directement métabolisée *in vivo* à la fois par les enzymes lipoxygénases et les enzymes cyclo-oxygénases,
- par "précurseur" de substrat pour lipoxygénases et cyclo-oxygénases, toute substance qui peut être métabolisée *in vivo* par l'organisme en un substrat convenable pour lipoxygénases et cyclo-oxygénases, ainsi que toute substance induisant la formation d'acides gras polyinsaturés dans les tissus vivants, ceci pouvant être objectivé par chromatographie gazeuse ou par toute autre technique standard comme celles décrites par Pelick et al. P23 "Analysis of lipids and lipoproteins" éditions Perkins American Oil Chemist Society, Champaign Illinois U.S.A.,
- par "inhibiteur" de lipoxygénases ou de cyclo-oxygénases, toute substance permettant, *in vivo*, de limiter ou d'inhiber totalement l'activité enzymatique de l'une ou l'autre de ces enzymes.
- par "stimulateur" ou "agoniste" de lipoxygénases ou de cyclo-oxygénases, toute substance permettant, *in vivo*, d'augmenter l'activité enzymatique de l'une ou de l'autre de ces enzymes, le terme "agoniste" devant être entendu par la suite comme inclus dans le terme général de "stimulateur",
- par "voie topique", toute technique d'administration d'un produit par application directe de ce dernier sur une partie superficielle (ou externe) du corps, telle que peau, cheveux et autres,
- par "voie systémique", toute technique d'administration d'un produit par une voie autre que topique, par exemple orale et/ou parentérale.

Ainsi, selon un premier aspect de la présente invention, il est maintenant proposé un procédé de traitement *in vivo* pour modifier la pousse des poils et/ou des cheveux, ledit procédé étant caractérisé par le fait qu'il consiste à administrer à l'organisme, par voie topique et/ou systémique, au moins un inhibiteur ou au moins un stimulateur ou agoniste de lipoxygénases ou de cyclo-oxygénases.

Dans le cas où le procédé de traitement ci-dessus est plus particulièrement destiné à favoriser la pousse et/ou limiter la chute des poils et/ou des cheveux, on met en oeuvre au moins une substance qui peut être un inhibiteur de lipoxygénases et/ou un stimulateur de cyclo-oxygénases.

Dans le cas où ledit procédé est cette fois plus particulièrement destiné à ralentir et/ou empêcher la pousse des poils et/ou des cheveux, on met en oeuvre au moins une substance qui peut être un stimulateur de lipoxygénases et/ou un inhibiteur de cyclo-oxygénases.

Selon un mode particulièrement préféré de mise en oeuvre du procédé de traitement selon l'invention, on administre en outre à l'organisme, à coté desdits inhibiteurs ou stimulateurs, au moins un substrat pour lipoxygénases et cyclo-oxygénases, ou un précurseur dudit substrat. L'apport dudit substrat, ou dudit précurseur, peut alors se faire par voie topique et/ou systémique, et ceci de manière simultanée, séparée, ou encore étalée dans le temps, par rapport à l'étape d'administration des inhibiteurs ou stimulateurs de lipoxygénases ou de cyclo-oxygénases.

Selon un autre aspect de la présente invention, il est également proposé des dispositifs à plusieurs compartiments ou "kits" destinés à la mise en oeuvre du procédé ci-dessus, et en particulier des kits qui sont caractérisés par le fait qu'ils comprennent dans un premier compartiment un ou plusieurs inhibiteurs, ou un ou plusieurs stimulateurs, de lipoxygénases ou de cyclo-oxygénases, et, dans un deuxième compartiment, un ou plusieurs substrats pour lipoxygénases et cyclo-oxygénases et/ou un ou plusieurs précurseurs desdits substrats, les compositions contenues dans lesdits premier et second compartiments étant ici considérées comme compositions de combinaison pour une utilisation simultanée, séparée ou étalée dans le temps dans un traitement destiné à modifier la pousse des poils et/ou des cheveux.

Selon enfin d'autres aspects de la présente invention, il est également proposé des compositions ou associations, nouvelles en soi, convenant à la mise en oeuvre du procédé selon l'invention pris dans ces diverses variantes.

Mais d'autres caractéristiques, aspects, objets et avantages de l'invention apparaitront encore plus clairement à la lecture de la description qui va suivre, ainsi que des divers exemples concrets, mais nullement limitatifs, destinés à l'illustrer.

On commencera par détailler, dans leur nature, les différents produits et compositions utilisables dans le cadre de la présente invention (inhibiteur/stimulateur/substrat/précurseur).

Le caractère inhibiteur ou stimulateur (ou agoniste) d'une substance donnée vis à vis des lipoxygénases ou des cyclo-oxygénases peut être classiquement déterminé par l'homme de l'art au moyen notamment de tests biochimiques usuels, généralement basés sur des méthodes d'analyses chromatographiques. Ainsi, ces activités peuvent être par exemple explorées avec les techniques suivantes (liste non limitative) ou avec toute autre technique standardisée :
- activités vis à vis des 5, 12 et 15 lipoxygénases : on peut ici citer la méthode consistant à utiliser un matériel biologique (polynucléaires humains, cheveux) incubé en présence d'acide arachidonique C14 ou d'acide linoléique C14; les hydroxyacides formés sont extraits, séparés par chromatographie sur couche mince ou chromatographie HPLC (Vanderhoeck J.Y. et Bailey J.M. dans J. Biol. Chem., 1984, 259, pp 6752-6761; Huang M. et al., dans Cancer Res., 51, pp 813-819, 1991; Baer A.N. et Green F.A. dans J. Lipids Res., 1993, 34, pp 1505-1514; Ziboh V.A. et al. dans J. invest. Dermatol., 83, pp 248-251, 1984).
- activités vis à vis de la 5 lipoxygénase : on peut ici citer les méthodes spectrophotométriques décrites par Aharony D. et Stein R.L. dans J.Biol.Chem., 1986, 261, pp 11512-11517, et par McMillan R.M. et al. dans Biochim. Biophys. Acta, 1989, 1005, pp 170-176.
- activités vis à vis des cyclo-oxygénases : on peut ici citer la méthode reposant sur l'utilisation d'un matériel biologique (épiderme) incubé en présence d'acide arachidonique C14; les hydroxyacides formés sont extraits, séparés par chromatographie HPLC (Huang M. et al. dans Cancer Res., 51, pp 813-819, 1991) ou identifiés par radioimmunoessais (Lysz T.W. et Needleman P. J., dans Neurochim., 38, pp 1111-1117, 1982). On peut également mentionner le test décrit dans l'article "Nitric Oxide Activates Cyclo-oxygénase Enzymes, par D. SALVAMENI et al., Proc. Natl. Sci. USA, Vol.90, pp 7240-7244, August 1993".

Les inhibiteurs de lipoxygénases peuvent être notamment choisis parmi les inhibiteurs redox et non redox, les précurseurs d'inhibiteurs redox, les agents anti-oxydants à l'exception des flavonoïdes, les agents chélateurs du fer, les composés imidazolés, les phénothiazines, les dérivés du benzopyrane, ainsi que parmi certains éicosanoïdes.

L'inhibiteur redox peut être choisi parmi les dérivés des butanes catécholiques (US 5 008 294, US 4 708 964 et US 4 880 637), tels que l'acide nordihydroguaiarétique (NDGA), ou l'un de ses énantiomères tel que le masoprocol.

L'inhibiteur redox peut aussi être choisi parmi la phénidone, le Ionapalen, les indazolinones, le naphazatrom, le benzofuranol, l'alkylhydroxylamine, les composés de formules suivantes :

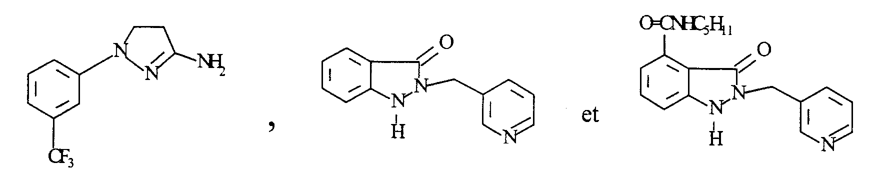

Les inhibiteurs non redox peuvent être choisis parmi les hydroxythiazoles, les méthoxyalkylthiazoles, les benzopyranes et leurs dérivés, le méthoxytétrahydropyrane, les acides boswelliques et leurs dérivés acétylés, les acides quinolinméthoxyphénylacétiques substitués par des radicaux cycloalkyles.

L'antioxydant peut être choisi parmi les phénols, le propylgallate.

On peut également citer les dérivés du chalcone tel que le 4,2',4'-trihydroxychalcone les orthoaminophénols, les N-hydroxyurées, les benzofuranols, l'ebselen et les agents susceptibles d'accroître l'activité des séléno-enzymes réductrices.

L'agent chélateur du fer peut être choisi parmi les acides hydroxamiques et leurs dérivés, les N-hydroxyurées, le 2-benzyl-1-naphtol, les catéchols, les hydroxylamines, le carnosol, le naphtol, la sulfasalazine, le zileuton, l'acide 5-hydroxyanthranilique et les acides 4-(ω-arylalkyl)phénylalcanoïques.

Le composé imidazolé peut être l'itraconazole.

Parmi les éicosanoïdes inhibiteurs de lipoxygénases, on peut citer les acides octadécatétraénoïque, éicosatétraénoïque, docosapenténoïque, eicosahexaénoïque, docosahexaénoïque, et leurs différents esters, de même que divers autres éicosanoïdes, éventuellement sous forme d'esters, tels que PGE1 (prostaglandineE1), PGA2 (prostaglandineA2), le viprostol, les acides 15monohydroxyéicosatétraénoïque, 15monohydroxyéicosatriénoïque et 15monohydroxyéicosapentaénoïque, les leukotriènes B5, C5 et D5.

A titre d'autres composés divers pouvant inhiber les lipoxygénases, on peut également citer les produits interférant avec les flux calciques, en particulier les phénothiazines et les diphénylbutylamines, le vérapamil, le fuscoside, le curcumin, l'acide chlorogénique, l'acide caféique, l'acide 5,8,11,14-éicosatétrayénoïque (ETYA), l'hydroxyphénylrétinamide, le Ionapalène, l'esculin, le diéthylcarbamazine, la phénantroline, la baicaléine, le proxicromil, les thioéthers et en particulier le sulfure de diallyle et le sulfure de di-(1 propènyl).

Les stimulateurs de lipoxygénases, quant à eux, peuvent être notamment choisis parmi les cytokines telles que le facteur de croissance des fibroblastes (FGFβ), le facteur de croissance tranformant (TGFβ) et le facteur de croissance épidermique (EGF).

Les inhibiteurs de cyclo-oxygénases peuvent être notamment choisis parmi les agents anti-inflammatoires non-stéroïdiens tels que les dérivés arylcarboxyliques, les dérivés pyrazolés, les dérivés de l'oxicam, les dérivés de l'acide nicotinique.

Les stimulateurs ou agonistes de cyclo-oxygénases peuvent être choisis notamment parmi les métabolites de l'acide arachidonique, le monoxyde d'azote et les composés donneurs de monoxyde d'azote, le stanozolol, les composés donneurs de glutathion, les neuropeptides et en particulier le peptide intestinal vasoactif (V.I.P.), les ionophores calciques, les anthocyanosides, tes bioflavonoïdes, la FGA, les facteurs activateurs de plaquettes (PAF).

Enfin, parmi les substances pouvant être utilisées à la fois comme inhibiteur des lipoxygénases et stimulateur des cyclo-oxygénases, on peut plus particulièrement citer la 6-chloro-2,3-dihydroxy-1,4-naphtoquinone (CNDQ).

Concernant maintenant les substrats convenables pour lipoxygénases et cyclo-oxygénases, on peut citer les acides gras polyinsaturés, en particulier ceux contenant 20 atomes de carbone, tels que l'acide arachidonique, l'acide dihomogamma-linolénique ou bien encore l'acide éicosapentaénoïque.

A titre de précurseurs de tels substrats, on peut plus particulièrement citer les acides gras polyinsaturés dits essentiels, tels que les acides linoléïque, a-linolénique et g-linolénique, ainsi que les phospholipides de la membrane cellulaire tels que la phosphatidylsérine, la phosphatidyléthanolamine, la phosphatidylcholine, le phosphatidylinositol, et le diphosphatidylglycérol.

Les substrats, ou précurseurs de substrats, ci-dessus, peuvent être obtenus notamment à partir de certains composés naturels, en particulier de certains aliments, d'origine animale, végétale ou microbienne (extraits d'huiles végétales telles que l'huile de Oénothéra biennis, l'huile de bourrache, l'huile de pépin de cassis, l'huile d'onagre, extraits d'huiles de poissons, extraits d'huiles de tissus d'insectes).

Selon l'invention, il est bien entendu possible d'utiliser directement de tels composés naturels qui contiennent les substrats et/ou les précurseurs de substrats désirés.
Il est également possible d'utiliser des produits obtenus par synthèse industrielle.

On notera enfin d'une manière générale que, dans le cadre de la présente invention, il est bien évidemment tout à fait possible d'utiliser des mélanges d'inhibiteurs, des mélanges de stimulateurs, des mélanges de substrats, des mélanges de précurseurs, ainsi que des mélanges de ces mélanges, pour autant bien entendu que ces mélanges de mélanges restent compatibles avec l'effet recherché.

On revient maintenant plus particulièrement à l'exposé du procédé selon l'invention.

Comme indiqué précédemment, ce procédé consiste essentiellement à administrer à l'organisme, par voie topique et/ou systémique, au moins un inhibiteur ou au moins un stimulateur de lipoxygénases ou de cyclo-oxygénases, ladite administration se faisant de préférence en combinaison (simultanée, séparée ou étalée dans le temps) avec au moins un substrat, ou un précurseur de substrat, tels qu'ils ont été définis ci-avant.

De manière préférentielle, les inhibiteurs et les stimulateurs d'enzymes sont administrés par voie topique.

Les substrats et/ou leurs précurseurs peuvent être, quant à eux, administrés soit par voie systémique, et dans ce cas de préférence par voie orale, soit plus préférentiellement encore par voie topique.

Selon un mode particulièrement préféré de mise en oeuvre du procédé selon l'invention, on applique sur la peau et/ou le cuir chevelu des compositions à usage topique contenant conjointement les inhibiteurs et/ou les stimulateurs d'enzymes d'une part et les substrats et/ou leurs précurseurs d'autre part.

D'une manière générale, on notera que tous les produits ci-dessus peuvent être classiquement conditionnés sous une forme convenant au mode d'administration ou d'application retenus pour ces derniers (lotions, shampooings, comprimés, sirops et autres).

Les compositions ou les "kits" rentrant dans le cadre de la présente invention sont plus particulièrement les suivants :
- les compositions (A) comprenant au moins un inhibiteur de lipoxygénases et au moins un stimulateur de cyclo-oxygénases,
- les compositions (B) comprenant au moins un inhibiteur de cyclo-oxygénases et au moins un stimulateur de lipoxygénases,
- les compositions (C) comprenant au moins un inhibiteur de lipoxygénases et/ou au moins un stimulateur de cyclo-oxygénases, en association avec au moins un substrat pour lipoxygénases et cyclo-oxygénases et/ou un précurseur d'un tel substrat,
- les compositions (D) comprenant au moins un inhibiteur de cyclo-oxygénases et/ou au moins un stimulateur de lipoxygénases, en association avec au moins un subtrat pour lipoxygénases et cyclo-oxygénases et/ou un précurseur d'un tel substrat,
- les "kits" (E) comprenant dans un premier compartiment au moins un inhibiteur de lipoxygénases et, dans un deuxième compartiment, au moins un stimulateur de cyclo-oxygénases,
- les "kits" (F) comprenant dans un premier compartiment au moins un inhibiteur de cyclo-oxygénases et, dans un deuxième compartiment, au moins un stimulateur de lipoxygénases,
- les "kits" (G) comprenant dans un premier compartiment au moins un inhibiteur de lipoxygénases et/ou au moins un stimulateur de cyclo-oxygénases ou inversement au moins un inhibiteur de cyclo-oxygénases et/ou au moins un stimulateur de lipoxygénases, et dans un deuxième compartiment au moins un substrat pour lipoxygénases et cyclo-oxygénases et/ou au moins un précurseur d'un tel substrat.

Comme indiqué précédemment, chacune des compositions (A), (B), (C) et (D), ainsi que chacun des composants rentrant dans les compartiments des kits (E), (F) et (G), sont classiquement conditionnés sous une forme convenant aux différents modes d'administration ou d'application envisagés pour ces derniers (lotions, shampooings, comprimés, sirops et autres). Ainsi, les compositions (A)-(D) et les kits (E)-(F) sont de préférence conditionnés sous une forme convenant à une application topique, et dans le cadre des kits (G), les composants du premier compartiment sont de préférence conditionnés sous une forme adaptée à une application topique, alors que les composants du deuxième compartiment sont conditionnés sous une forme adaptée à une administration par voie orale.

D'une manière générale, selon la présente invention, il est en fait possible de concevoir des kits de présentation contenant autant de compartiments séparés que de substances (inhibiteurs, stimulateurs, substrats, précurseurs de substrats) que l'on désire ou qu'il convient de mettre en oeuvre.

Les compositions selon l'invention, ou les kits selon l'invention, ou la mise en oeuvre du procédé selon l'invention, peuvent également faire appel à divers additifs classiques et usuels, en particulier cosmétiques dans le cas d'applications topiques (produits capillaires notamment), choisis par exemple parmi des filtres UV, des agents épaississants, des agents de pénétration tel que l'urée, des solvants organiques tels que l'éthanol, l'isopropanol, les alkylèneglycols, des agents tensio-actifs choisis parmi les tensio-actifs non-ioniques tels que les alkylpolyglycosides, les tensio-actifs cationiques, les tensio-actifs anioniques et les tensio-actifs amphotères, des colorants, des agents anti-pelliculaires, des parfums et des conservateurs.

Il est également possible d'intégrer dans les compositions selon l'invention des produits déja connus en soi comme présentant une activité dans le domaine de la pousse des poils et/ou des cheveux, comme par exemple le 2,4-diamino-6-pipéridinopyrimidine-3-oxyde notamment commercialisé sous le nom de marque "Minoxidil" par la Société UPJOHN.

Pour obtenir des effets notables, la fréquence d'administration ou d'application des compositions selon l'invention, tant avec que sans substrat ou précurseur de substrat, est de l'ordre de une à deux fois par jour. A cet égard, on a noté que les quantités suffisantes en agents inhibiteurs et/ou stimulateurs à mettre en oeuvre dans le cadre de l'invention peuvent, généralement, rester très faibles.

La présente invention trouve des applications particulièrement utiles dans le domaine des traitements des diverses pathologies affectant la peau et/ou le cuir chevelu, en particulier l'hirsutisme et les alopécies, notamment les alopécies iatrogènes.

Des exemples concrets illustrant l'invention vont maintenant être donnés.

| **Exemple 1: Lotion non rincée** | |
|---|---|
| NDGA | 0,1 g |
| Acide linoléique | 0,1 g |
| Propylène glycol | 22,8 g |
| Ethanol 95° | 55,1 g |
| Eau purifiée | qsp 100 g |

| **Exemple 2 : Lotion non rincée** | |
|---|---|
| NDGA | 2 g |
| Acide linoléique | 5 g |
| Propylène glycol | 22,8 g |
| Ethanol 95° | 55,1 g |
| Eau purifiée | qsp 100 g |

| **Exemple 3 : Lotion rincée** | |
|---|---|
| NDGA | 5 g |
| Acide linoléique | 5 g |
| Propylène glycol | 22,8 g |
| Ethanol 95° | 55,1 g |
| Eau purifiée | qsp 100 g |

| **Exemple 4 : Lotion rincée** | |
|---|---|
| NDGA | 8 g |
| Acide linoléique | 15 g |
| Propylène glycol | 22,8 g |
| Ethanol absolu | qsp 100 g |

| **Exemple 5 : Shampooing** | |
|---|---|
| NDGA | 1 g |
| Acide linoléique | 1 g |
| Tensio-actif APG 300 | 15 g MA (= 30 g) |
| Eau purifiée | qsp 100 g |

| **Exemple 6 : Lotion non rincée** | |
|---|---|
| Gingko Biloba ⁽¹⁾ | 5 g |
| Propylène glycol | 22,8 g |
| Ethanol 95° | 55,1 g |
| Eau purifiée | qsp 100 g |

| | |
|---|---|
| ⁽¹⁾ : extrait naturel riche en flavonoïdes | |

| **Exemple 7: Lotion non rincée** | |
|---|---|
| Kétokonazole | 0,5 g |
| Propylène glycol | 22,8 g |
| Ethanol 95° | 55,1 g |
| Eau purifiée | qsp 100 g |

| **Exemple 8: Lotion non rincée** | |
|---|---|
| Sulfure de diallyle | 11,4 g |
| Propylène glycol | 22,8 g |
| Ethanol 95° | 55,1 g |
| Eau purifiée | qsp 100 g |

| **Exemple 9: Lotion non rincée** | |
|---|---|
| Gingko Biloba | 5 g |
| Acide linoléique | 5 g |
| Propylène glycol | 22,8 g |
| Ethanol 95° | 55,1 g |
| Eau purifiée | qsp 100 g |

| **Exemple 10: Lotion non rincée** | |
|---|---|
| Kétokonazole | 0,5 g |
| Acide linoléique | 5 g |
| Propylène glycol | 22,8 g |
| Ethanol 95° | 55,1 g |
| Eau purifiée | qsp 100 g |

| **Exemple 11: Lotion non rincée** | |
|---|---|
| Sulfure de diallyle | 11,4 g |
| Acide linoléique | 5 g |
| Propylène glycol | 22,8 g |
| Ethanol 95° | 55,1 g |
| Eau puriifiée | qsp 100 g |

| **Exemple 12: Lotion non rincée** | |
|---|---|
| Gingko Biloba | 5 g |
| Huile de bourrache ⁽²⁾ | 10 g |
| Propylène glycol | 22,8 g |
| Ethanol 95° | 55,1 g |
| Eau purifiée | qsp 100 g |

| | |
|---|---|
| (2) : extrait naturel contenant des acides gras polyinsaturés 18:2n-6 et 18:3n-6 | |

| **Exemple 13: Lotion non rincée** | |
|---|---|
| CDNQ | 2 g |
| Acide linoléique | 5 g |
| Propylène glycol | 22,8 g |
| Ethanol 95° | 55,1 g |
| Eau puriifiée | qsp 100 g |

| **Exemple 14: Lotion non rincée** | |
|---|---|
| CDNQ | 2 g |
| Huile de bourrache | 10 g |
| Propylène glycol | 22,8 g |
| Ethanol 95° | 55,1 g |
| Eau purifiée | qsp 100 g |

| **Exemple 15: Lotion non rincée** | |
|---|---|
| Indométacine | 0,25g |
| Propylène glycol | 22,8 g |
| Ethanol 95° | 55,1 g |
| Eau purifiée | qsp 100 g |

| **Exemple 16: Lotion non rincée** | |
|---|---|
| Indométacine | 0,25g |
| Acide docosahexaénoïque | 2 g |
| Propylène glycol | 22,8 g |
| Ethanol 95° | 55,1 g |
| Eau purifiée | qsp 100 g |

Toutes les compositions 1 à 14 ci-dessus ont donné de bons résultats dans la pousse des poils et des cheveux, en particulier celles contenant un substrat ou un précurseur de substrat pour lipoxygénases et cyclo-oxygénases. Les exemples 15 et 16 ont, quant à eux, donné de bons résultats dans le ralentissement de la pousse des poils et des cheveux.

## Revendications

1. Procédé de traitement cosmétique non thérapeutique pour favoriser la pousse et/ou limiter la chute des poils et/ou des cheveux, caractérisé en ce que l'on utilise au moins un inhibiteur de lipoxygénases à l'exception du kétoconazole ou des flavonoïdes et/ou au moins un stimulateur de cyclooxygénases.

2. Procédé selon la revendication 1, caractérisé en ce que l'on utilise au moins un inhibiteur de lipoxygénases et au moins un stimulateur de cyclooxygénases.

3. Procédé selon la revendication 2, caractérisé en ce que l'on utilise au moins une substance qui est à la fois un inhibiteur de lipoxygénases et un stimulateur de cyclooxygénases.

4. Procédé de traitement cosmétique non thérapeutique pour ralentir et/ou empêcher la pousse des poils et/ou des cheveux, caractérisé en ce que l'on utilise au moins un stimulateur de lipoxygénases et au moins un inhibiteur de cyclooxygénases.

5. Procédé selon la revendication 4, caractérisé en ce que l'on utilise au moins une substance qui est à la fois un stimulateur de lipoxygénases et un inhibiteur de cyclooxygénases.

6. Procédé selon l'une quelconque des revendications précédentes, caractérisé en ce que les inhibiteurs et les stimulateurs de lipoxygénases ou de cyclooxygénases sont administrés par voie topique.

7. Procédé selon l'une quelconque des revendications précédentes, caractérisé en ce que l'on apporte en outre à l'organisme, par voie topique et/ou systémique, au moins un substrat pour lipoxygénases et cyclooxygénases et/ou au moins un précurseur de substrat pour lipoxygénases et cyclooxygénases.

8. Procédé selon la revendication 7, caractérisé en ce que ledit apport de substrat ou de précurseur de substrat se fait de manière simultanée, séparée ou étalée dans le temps, par rapport à l'étape d'administration des inhibiteurs ou des stimulateurs de lipoxygénases ou de cyclooxygénases.

9. Procédé selon l'une des revendications 7 ou 8, caractérisé en ce que ledit apport se fait par voie orale et/ou topique.

10. Procédé selon la revendication 9, caractérisé en ce que ledit apport se fait par voie topique.

11. Procédé selon la revendications 10, caractérisé en ce qu'il consiste à utiliser une composition à usage topique contenant à la fois les inhibiteurs ou stimulateurs de lipoxygénases ou de cyclooxygénases d'une part et les substrats ou précurseurs de substrats pour lipoxygénases et cyclooxygénases d'autre part.

12. Procédé selon l'une quelconque des revendications précédentes, caractérisé en ce que l'on utilise des inhibiteurs de lipoxygénases qui sont choisis parmi les inhibiteurs redox et non redox, les précurseurs d'inhibiteurs redox, les agents anti-oxydants, les agents chélateurs du fer, les composés imidazolés, les phénothiazines, les dérivés du benzopyrane, les éicosanoïdes inhibiteurs, les produits interférant avec les flux calciques et en particulier les phénothiazines et les diphénylbutylamines, le vérapamil, le fuscoside, le curcumin, l'acide chlorogénique, l'acide cafféique, l'acide 5,8,11,14-éicosatétrayénoïque (ETYA), l'hydroxyphénylrétinamide, le Ionapalène, l'esculin, le diéthylcarbamazine, la phénantroline, la baicaléine, le proxicromil, les thioéthers et en particulier le sulfure de diallyle et le sulfure de di-(1 propényl).

13. Procédé selon la revendication 12, caractérisé en ce que les inhibiteurs redox sont choisi parmi les dérivés des butanes catécholiques.

14. Procédé selon la revendication 13, caractérisé en ce que les dérivés des butanes catécholiques sont choisis parmi l'acide nordihydroguaiarétique (NDGA), le masoprocol.

15. Procédé selon la revendication 12, caractérisé en ce que les inhibiteurs redox sont choisis parmi la phénidone, le lanopalen, les indazolinones, le naphazatrom, le benzofuranol, l'alkylhydroxylamine, les composés de formules suivantes:

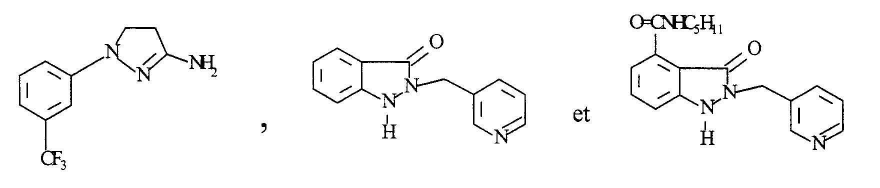

16. Procédé selon la revendication 12, caractérisé en ce que les inhibiteurs non redox sont choisis parmi les hydroxythiazoles, les méthoxyalkylthiazoles, les benzopyranes et leurs dérivés, le méthoxytétrahydropyrane, les acides boswelliques et leurs dérivés acétylés, les acides quinolinméthoxyphénylacétiques substitués par des radicaux cycloalkyles.

17. Procédé selon la revendication 12, caractérisé en ce que les agents anti-oxydants sont choisis parmi les phénols, le propylgallate, les flavonoïdes et/ou les composés naturels contenant de tels flavonoïdes, en particulier les dérivés hydroxylés des flavones tels que le flavonol, la dihydroquercétine, la lutéoline, la galangine, l'orobol, les dérivés du chalcone tel que le 4,2',4'-trihydroxychalcone, les orthoaminophénols, les N-hydroxyurées, les benzofuranols, l'ebselen et les agents susceptibles d'accroître l'activité des séléno-enzymes réductrices.

18. Procédé selon la revendication 12, caractérisé les agents chélateurs du fer sont choisis parmi les acides hydroxamiques et leurs dérivés, les N-hydroxyurées, le 2-benzyl-1-naphtol, les catéchols, les hydroxylamines, le carnosol, le naphtol, la sulfasalazine, le zileuton, l'acide 5-hydroxyanthranilique et les acides 4-(ω-arylalkyl)phénylalcanoïques.

19. Procédé selon la revendication 12, caractérisé en ce que les composés imidazolés est l'itraconazole.

20. Procédé selon la revendication 12, caractérisé en ce que les éicosanoïdes inhibiteurs sont choisis parmi les acides octa-décatétraénoïque, éicosatétraénoïque, docosapenténoïque, eicosahexaénoïque, docosahexaénoïque, et leurs différents esters, de même que divers autres éicosanoïdes, éventuellement sous forme d'esters, tels que PGE1 (prostaglandineE1), PGA2 (prostaglandineA2), le viprostol, les acides 15monohydroxyéicosatétraénoique, 15monohydroxyéicosatriénoique et 15monohydroxyéicosapentaénoique, les leukotriènes B5, C5 et D5.

21. Procédé selon l'une quelconque des revendications précédentes, caractérisé en ce que l'on utilise des inhibiteurs de cyclooxygénases qui sont choisis parmi les agents anti-inflammatoires non stéroïdiens.

22. Procédé selon la revendication 21, caractérisé en ce que les agents anti-inflammatoires non stéroïdiens sont choisis parmi les dérivés arylcarboxyliques, les dérivés pyrazolés, les dérivés de l'oxicam, les dérivés de l'acide nicotinique.

23. Procédé selon l'une quelconque des revendications précédentes, caractérisé en ce que l'on utilise des stimulateurs ou agonistes de cyclooxygénases qui sont choisis parmi les métabolites de l'acide arachidonique, le monoxyde d'azote et les composés donneurs de monoxyde d'azote, le stanozolol, les composés donneurs de glutathion, les neuropeptides et en particulier le V.I.P., les ionophores calciques, les anthocyanosides, les bioflavonoïdes, la FGA, les facteurs activateurs de plaquettes (PAF).

24. Procédé selon l'une quelconque des revendications précédentes, caractérisé en ce que l'on utilise des stimulateurs de lipoxygénases qui sont choisis parmi les cytokines telles que le FGFβ, le TGFβ et le facteur de croissance épidermique (EGF).

25. Procédé selon l'une quelconque des revendications précédentes, caractérisé en ce que l'on utilise à titre de substance agissant à la fois comme inhibiteur des lipoxygénases et stimulateur des cyclooxygénases, la 6-chloro-2,3-dihydroxy-1,4-naphtoquinone (CNDQ).

26. Procédé selon l'une quelconque des revendications 7 à 25, caractérisé en ce que l'on utilise des substrats pour lipoxygénases et cyclooxygénases qui sont choisis parmi les acides gras polyinsaturés, en particulier ceux contenant 20 atomes de carbone.

27. Procédé selon la revendication 26, caractérisé en ce que lesdits acides gras polyinsaturés sont choisis, seuls ou en mélanges, parmi l'acide arachidonique, l'acide dihomo-γ-linolénique et l'acide éicosapentaénoïque.

28. Procédé selon l'une quelconque des revendications 7 à 27, caractérisé en ce que l'on utilise des précurseurs de substrats pour lipoxygénases et cyclooxygénases qui sont choisis parmi les acides gras essentiels et les phospholipides de la membrane cellulaire.

29. Procédé selon la revendication 28, caractérisé en ce que lesdits acides gras essentiels sont choisis, seuls ou en mélanges, parmi les acides linoléique, α-linolénique et β-linolénique.

30. Procédé selon la revendication 28, caractérisé en ce que lesdits phospholipides sont choisis, seuls ou en mélanges, parmi la phosphatidylsérine, la phosphatidyléthanolamine, la phosphatidylcholine, le phosphatidylinositol et le diphosphatidylglycérol.

31. Compositions, caractérisées en ce qu'elles comprennent au moins un inhibiteur de lipoxygénases associé à au moins un stimulateur de cyclooxygénases ou inversement au moins un inhibiteur de cyclooxygénase associé à au moins un stimulateur de lipoxygénases.

32. Compositions, caractérisées en ce qu'elles comprennent au moins un substrat pour lipoxygénases et cyclooxygénases et/ou un précurseur d'un substrat pour lipoxygénases et cyclooxygénases, en association avec au moins un inhibiteur de lipoxygénases et/ou au moins un stimulateur de cyclooxygénases ou inversement au moins un inhibiteur de cyclooxygénases et/ou au moins un stimulateur de lipoxygénases.

33. Compositions selon l'une des revendications 31 ou 32, caractérisées en ce qu'elles sont conditionnées sous une forme convenant à une application topique.

34. Compositions selon l'une quelconque des revendications 31 à 33, caractérisées en ce que les inhibiteurs, les stimulateurs, les substrats et les précurseurs de substrats des enzymes lipoxygénases et cyclooxygénases sont tels que définis aux revendications 12 à 30.

35. Dispositifs à plusieurs compartiments ou "kits", caractérisés en ce qu'ils comprennent dans un premier compartiment au moins un inhibiteur de lipoxygénases, ou inversement au moins un inhibiteur de cyclooxygénases, et, dans un deuxième compartiment, au moins un stimulateur de cyclooxygénases, ou inversement au moins stimulateur de lipoxygénases.

36. "Kits" selon la revendication 35, caractérisés en ce que les composés rentrant dans lesdits premier et deuxième compartiments sont conditionnés sous une forme convenant à une application topique.

37. Dispositifs à plusieurs compartiments ou "kits", caractérisés en ce qu'ils comprennent dans un premier compartiment au moins un inhibiteur de lipoxygénases et/ou au moins un stimulateur de cyclooxygénases ou inversement au moins un inhibiteur de cyclooxygénases et/ou au moins un stimulateur de lipoxygénases, et dans un deuxième compartiment au moins un substrat pour lipoxygénases et cyclooxygénases et/ou un précurseur d'un substrat pour lipoxygénases et cyclooxygénases.

38. "Kits" selon la revendication 37, caractérisés en ce que les composés rentrant dans ledit premier compartiment sont conditionnés sous une forme convenant à une application topique, et les composés rentrant dans ledit deuxième compartiment sont conditionnés sous une forme convenant à une administration par voie orale ou une application par voie topique.

39. "Kits" selon l'une des revendications 37 ou 38, caractérisés en ce que les inhibiteurs, les stimulateurs, les substrats et les précurseurs de substrats des enzymes lipoxygénases et cyclooxygénases sont tels que définis aux revendications 14 à 32.

40. Utilisation non-thérapeutique d'un inhibiteur de lipoxygénases à l'exception du kétoconazole et des flavonoïdes et/ou d'un stimulateur de cyclooxygénases, comme agent(s) pour favoriser la pousse et/ou limiter la chute des poils et/ou des cheveux.

41. Utilisation non-thérapeutique d'un stimulateur de lipoxygénases et d'un inhibiteur de cyclooxygénases, comme agent(s) pour ralentir et/ou empêcher la pousse des poils et/ou des cheveux.

42. Utilisation selon l'une des revendications 40 ou 41, caractérisée en ce que lesdits inhibiteurs ou stimulateurs de lipoxygénases ou de cyclooxygénases sont mis en oeuvre au moyen de compositions ou de "kits" tels que définis à l'une quelconque des revendications 31 à 39.

## Claims

1. Non-therapeutic cosmetic treatment process for promoting the growth and/or limiting the loss of body and/or head hair, characterized in that at least one lipoxygenase inhibitor, with the exception of ketoconazole or flavonoids, and/or at least one cyclooxygenase stimulator is used.

2. Process according to Claim 1, characterized in that at least one lipoxygenase inhibitor and at least one cyclooxygenase stimulator are used.

3. Process according to Claim 2, characterized in that at least one substance is used which is both a lipoxygenase inhibitor and a cyclooxygenase stimulator.

4. Non-therapeutic cosmetic treatment process for slowing down and/or preventing the growth of body and/or head hair, characterized in that at least one lipoxygenase stimulator and at least one cyclooxygenase inhibitor are used.

5. Process according to Claim 4, characterized in that at least one substance is used which is both a lipoxygenase stimulator and a cyclooxygenase inhibitor.

6. Process according to any one of the preceding claims, characterized in that the lipoxygenase or cyclooxygenase inhibitors and stimulators are administered via the topical route.

7. Process according to any one of the preceding claims, characterized in that at least one substrate for lipoxygenases and cyclooxygenases and/or at least one precursor of a substrate for lipoxygenases and cyclooxygenases is additionally supplied to the organism, via the topical and/or systemic route.

8. Process according to Claim 7, characterized in that the said supply of substrate or of precursor of a substrate is effected simultaneously, separately or sequentially, relative to the step of administration of the lipoxygenase or cyclooxygenase inhibitors or stimulators.

9. Process according to either of Claims 7 and 8, characterized in that the said supply is effected via the oral and/or topical route.

10. Process according to Claim 9, characterized in that the said supply is effected via the topical route.

11. Process according to Claim 10, characterized in that it consists in using a composition for topical use containing both the lipoxygenase or cyclooxygenase inhibitors or stimulators on the one hand, and the substrates or precursors of substrates for lipoxygenases and cyclooxygenases on the other hand.

12. Process according to any one of the preceding claims, characterized in that lipoxygenase inhibitors are used which are chosen from redox and non-redox inhibitors, redox inhibitor precursors, antioxidants, iron-chelating agents, imidazole-containing compounds, phenothiazines, benzopyran derivatives, inhibitory eicosanoids, products interfering with the flow of calcium and in particular phenothiazines and diphenylbutylamines, verapamil, fuscoside, curcumin, chlorogenic acid, caffeic acid, 5,8,11,14-eicosatetraynoic acid (ETYA), hydroxyphenylretinamide, lonapalene, esculin, diethylcarbamazine, phenanthroline, baicalein, proxicromil, thioethers and in particular diallyl sulphide and di-(1-propenyl) sulphide.

13. Process according to Claim 12, characterized in that the redox inhibitors are chosen from catecholbutane derivatives.

14. Process according to Claim 13, characterized in that the catecholbutane derivatives are chosen from nordihydroguaiaretic acid (NDGA) and masoprocol.

15. Process according to Claim 12, characterized in that the redox inhibitors are chosen from phenidone, lanopalene, indazolinones, naphazatrom, benzofuranol, alkylhydroxylamine and the compounds of the following formulae:

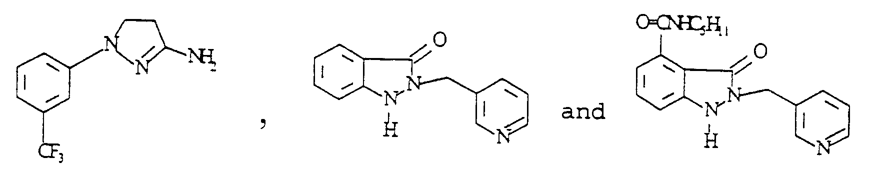

16. Process according to Claim 12, characterized in that the non-redox inhibitors are chosen from hydroxythiazoles, methoxyalkylthiazoles, benzopyrans and the derivatives thereof, methoxytetrahydropyran, boswellic acids and the acetyl derivatives thereof, and quinolinemethoxyphenylacetic acids substituted with cycloalkyl radicals.

17. Process according to Claim 12, characterized in that the antioxidants are chosen from phenols, propyl gallate, flavonoids and/or natural compounds containing such flavonoids, in particular hydroxylated flavone derivatives such as flavonol, dihydroquercetin, luteolin, galangin and orobol, chalcone derivatives such as 4,2',4'-trihydroxychalcone, ortho-aminophenols, N-hydroxyureas, benzofuranols and ebselen, and agents capable of enhancing the activity of reducing selenoenzymes.

18. Process according to Claim 12, characterized in that the iron-chelating agents are chosen from hydroxamic acids and derivatives thereof, N-hydroxyureas, 2-benzyl-1-naphthol, catechols, hydroxylamines, carnosol, naphthol, sulphasalazine, zyleuton, 5-hydroxyanthranilic acid and 4-(ω-arylalkyl)phenylalkanoic acids.

19. Process according to Claim 12, characterized in that the imidazole-containing compounds are itraconazole.

20. Process according to Claim 12, characterized in that the inhibitory eicosanoids are chosen from octadecatetraenoic acid, eicosatetraenoic acid, docosapentenoic acid, eicosahexaenoic acid and docosahexaenoic acid and the various esters thereof, as well as various other eicosanoids, which may optionally be in ester form, such as PGE₁ (prostaglandin E₁), PGA₂ (prostaglandin A₂), viprostol, 15-monohydroxyeicosatetraenoic acid, 15-monohydroxyeicosatrienoic acid and 15-monohydroxyeicosapentaenoic acid and leukotrienes B5, C5 and D5.

21. Process according to any one of the preceding claims, characterized in that cyclooxygenase inhibitors are used which are chosen from non-steroidal anti-inflammatory agents.

22. Process according to Claim 21, characterized in that the non-steroidal anti-inflammatory agents are chosen from arylcarboxylic derivatives, pyrazole-containing derivatives, oxicam derivatives and nicotinic acid derivatives.

23. Process according to any one of the preceding claims, characterized in that cyclooxygenase stimulators or agonists are used which are chosen from arachidonic acid metabolites, nitric oxide and nitric oxide-donating compounds, stanozolol, glutathione-donating compounds, neuropeptides and in particular V.I.P., calcium ionophores, anthocyanosides, bioflavonoids, FGA and platelet activating factors (PAF).

24. Process according to any one of the preceding claims, characterized in that lipoxygenase stimulators are used which are chosen from cytokines such as FGFβ, TGFβ and epidermal growth factor (EGF).

25. Process according to any one of the preceding claims, characterized in that 6-chloro-2,3-dihydroxy-1,4-naphthoquinone (CNDQ) is used as substance acting both as lipoxygenase inhibitor and cyclooxygenase stimulator.

26. Process according to any one of Claims 7 to 25, characterized in that substrates for lipoxygenases and cyclooxygenases are used which are chosen from polyunsaturated fatty acids, in particular those containing 20 carbon atoms.

27. Process according to Claim 26, characterized in that the said polyunsaturated fatty acids are chosen, alone or as mixtures, from arachidonic acid, dihomo-γ-linolenic acid and ecosapentaenoic acid.

28. Process according to any one of Claims 7 to 27, characterized in that precursors of substrates for lipoxygenases and cyclooxygenases are used which are chosen from essential fatty acids and cell membrane phospholipids.

29. Process according to Claim 28, characterized in that the said essential fatty acids are chosen, alone or as mixtures, from linoleic, α-linolenic and β-linolenic acids.

30. Process according to Claim 28, characterized in that the said phospholipids are chosen, alone or as mixtures, from phosphatidylserine, phosphatidylethanolamine, phosphatidylcholine, phosphatidylinositol and diphosphatidylglycerol.

31. Compositions, characterized in that they comprise at least one lipoxygenase inhibitor combined with at least one cyclooxygenase stimulator or, conversely, at least one cyclooxygenase inhibitor combined with at least one lipoxygenase stimulator.

32. Compositions, characterized in that they comprise at least one substrate for lipoxygenases and cyclooxygenases and/or one precursor of a substrate for lipoxygenases and cyclooxygenases, in combination with at least one lipoxygenase inhibitor and/or at least one cyclooxygenase stimulator or, conversely, at least one cyclooxygenase inhibitor and/or at least one lipoxygenase stimulator.

33. Compositions according to either of Claims 31 and 32, characterized in that they are packaged in a form suitable for topical application.

34. Compositions according to any one of Claims 31 to 33, characterized in that the inhibitors, the stimulators, the substrates and the precursors of substrates for the lipoxygenase and cyclooxygenase enzymes are as defined in Claims 12 to 30.

35. Devices containing several compartments or "kits", characterized in that they comprise, in a first compartment, at least one lipoxygenase inhibitor or, conversely, at least one cyclooxygenase inhibitor and, in a second compartment, at least one cyclooxygenase stimulator or, conversely, at least one lipoxygenase stimulator.

36. "Kits" according to Claim 35, characterized in that the compounds entering into the said first and second compartments are packaged in a form suitable for topical application.

37. Devices containing several compartments or "kits", characterized in that they comprise, in a first compartment, at least one lipoxygenase inhibitor and/or at least one cyclooxygenase stimulator or, conversely, at least one cyclooxygenase inhibitor and/or at least one lipoxygenase stimulator and, in a second compartment, at least one substrate for lipoxygenases and cyclooxygenases and/or a precursor of a substrate for lipoxygenases and cyclooxygenases.

38. "Kits" according to Claim 37, characterized in that the compounds entering into the said first compartment are packaged in a form suitable for topical application, and the compounds entering into the said second compartment are packaged in a form suitable for administration via the oral route or administration via the topical route.

39. "Kits" according to either of Claims 37 and 38, characterized in that the inhibitors, the stimulators, the substrates and the precursors of substrates for the lipoxygenase and cyclooxygenase enzymes are as defined in Claims 14 to 32.

40. Non-therapeutic use of a lipoxygenase inhibitor, with the exception of ketoconazole and flavonoids, and of a cyclooxygenase stimulator as agent(s) for promoting the growth and/or limiting the loss of body and/or head hair.

41. Non-therapeutic use of a lipoxygenase stimulator and of a cyclooxygenase inhibitor as agent(s) for slowing down and/or preventing the growth of body and/or head hair.

42. Use according to either of Claims 40 and 41, characterized in that the said lipoxygenase or cyclooxygenase inhibitors or stimulators are implemented by means of compositions or "kits" as defined in any one of Claims 31 to 39.

## Patentansprüche

1. Kosmetisches, nicht therapeutisches Behandlungsverfahren, um das Wachstum der Körperhaare und/oder der Haare zu begünstigen und/oder den Haarausfall zu beschränken, dadurch gekennzeichnet, daß mindestes ein Lipoxygenase-Inhibitor mit Ausnahme von Ketoconazol oder Flavonoiden und/oder mindestens ein Cyclooxygenase-Stimulator verwendet wird.

2. Verfahren nach Anspruch 1, dadurch gekennzeichnet, daß mindestes ein Lipoxygenase-Inhibitor und mindestens ein Cyclooxygenase-Stimulator verwendet wird.

3. Verfahren nach Anspruch 2, dadurch gekennzeichnet, daß mindestes eine Substanz verwendet wird, die gleichzeitig ein Lipoxygenase-Inhibitor und ein Cyclooxygenase-Stimulator ist.

4. Kosmetisches, nicht therapeutisches Behandlungsverfahren, um das Wachstum der Körperhaare und/oder Haare zu verlangsamen und/oder zu unterbinden, dadurch gekennzeichnet daß mindestens ein Lipoxygenase-Stimulator und mindestens ein Cyclooxygenase-Inhibitor verwendet wird.

5. Verfahren nach Anspruch 4, dadurch gekennzeichnet, daß mindestes eine Substanz verwendet wird, die gleichzeitig ein Lipoxygenase-Stimulator und ein Cyclooxygenase-Inhibitor ist.

6. Verfahren nach einem der vorhergehenden Ansprüche, dadurch gekennzeichnet, daß die Inhibitoren und Stimulatoren von Lipoxygenasen oder Cyclooxygenasen auf topischem Wege verabreicht werden.

7. Verfahren nach einem der vorhergehenden Ansprüche, dadurch gekennzeichnet, daß dem Organismus ferner auf topischem und/oder systemischem Wege mindestens ein Substrat für Lipoxygenasen und Cyclooxygenasen und/oder mindestens eine Vorläuferverbindung eines Substrats für Lipoxygenasen und Cyclooxygenasen verabreicht wird.

8. Verfahren nach Anspruch 7, dadurch gekennzeichnet, daß das Substrat oder die Vorläuferverbindung in Bezug auf die Verabreichung der Inhibitoren und Stimulatoren von Lipoxygenasen oder Cyclooxygenasen simultan, getrennt oder zeitlich verschoben verabreicht wird.

9. Verfahren nach einem der Ansprüche 7 oder 8, dadurch gekennzeichnet, daß die Verabreichung oral und/oder topisch erfolgt.

10. Verfahren nach Anspruch 9, dadurch gekennzeichnet, daß die Verabreichung topisch erfolgt.

11. Verfahren nach Anspruch 10, dadurch gekennzeichnet, daß es darin besteht, eine Zusammensetzung zur topischen Anwendung zu verwenden, die gleichzeitig einerseits die Inhibitoren oder Stimulatoren der Lipoxygenasen oder Cyclooxygenasen und andererseits die Substrate und/oder Vorläuferverbindungen der Substrate für Lipoxygenasen und Cyclooxygenasen enthält.

12. Verfahren nach einem der vorhergehenden Ansprüche, dadurch gekennzeichnet, daß Lipoxygenase-Inhibitoren verwendet werden, die unter den Redox-Inhibitoren, Nicht-Redox-Inhibitoren Vorläufern von Redox-Inhibitoren, Antioxidantien, Eisen-Chelatbildnern, Imidazol-haltigen Verbindungen, Phenothiazinen, Benzopyranderivaten, Eicosanoid-Inhibitoren, Produkten, die den Calcium-Fluß beeinflussen, insbesondere Phenothiazinen und Diphenylbutylaminen, Verapamil, Fuscoside, Curcumin, Chlorogensäure, Kaffeesäure, 5,8,11,14-Eicosatetraiensäure (ETYA), Hydroxyphenylretinamid, Lonapalene, Aesculin, Diethylcarbamazin, Phenantrolin, Baicalein, Proxicromil und den Thioethern, insbesondere Diallysulfid und Di-(1-propenyl)-sulfid, ausgewählt sind.

13. Verfahren nach Anspruch 12, dadurch gekennzeichnet, daß die Redox-Inhibitoren unter den Catechinbutanderivaten ausgewählt sind.

14. Verfahren nach Anspruch 13, dadurch gekennzeichnet, daß die Catechinbutanderivate unter Nordihydroguajaretsäure (NDGA) und Masoprocol ausgewählt sind.

15. Verfahren nach Anspruch 12, dadurch gekennzeichnet, daß die Redox-Inhibitoren unter Phenidone, Lonapalene, Indazolinonen, Nafazatrom, Benzofuranol, Alkylhydroxylamin und den folgenden Verbindungen ausgewählt sind:

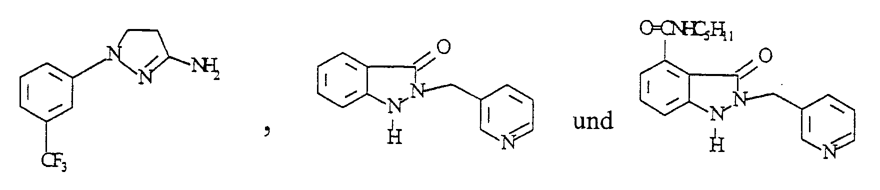

16. Verfahren nach Anspruch 12, dadurch gekennzeichnet, daß die Nicht-Redox-Inhibitoren unter Hydroxythiazolen, Methoxyalkylthiazolen, Benzopyranen und ihren Derivaten, Methoxytetrahydropyran, Boswellinsäuren und ihren acetylierten Derivaten und mit Cycloalkylgruppen substituierten Chinolinmethoxyphenylessigsäuren ausgewählt sind.

17. Verfahren nach Anspruch 12, dadurch gekennzeichnet, daß die Antioxidationsmittel unter den Phenolen, Propylgallat, Flavonoiden und/oder natürlichen Substanzen, die Flavonoide enthalten, und insbesondere hydroxylierten Flavonderivaten, wie Flavonol, Dihydroquercetin, Luteolin, Galangin, Orobol, Chalkonderivaten, wie 4,2',4'-Trihydroxychalkon, ortho-Aminophenolen, N-Hydroxyharnstoffen, Benzofuranolen, Ebselen und Mitteln, die die Aktivität von reduzierenden Seleno-Enzymen steigern können, ausgewählt sind.

18. Verfahren nach Anspruch 12, dadurch gekennzeichnet, daß die Eisen-Chelatbildner unter den Hydroxamsäuren und ihren Derivaten, N-Hydroxyharnstoffen, 2-Benzyl-1-naphthol, Catechinen, Hydroxylaminen, Carnosol, Naphthol, Sulfasalazin, Zileuton, 5-Hydroxyanthranilsäure und 4-(ω-Arylalkyl)-phenyl-alkansäuren ausgewählt sind.

19. Verfahren nach Anspruch 12, dadurch gekennzeichnet, daß die Imidazol-haltige Verbindung das Itraconazol ist.

20. Verfahren nach Anspruch 12, dadurch gekennzeichnet, daß die Eicosanoid-Inhibitoren unter Octadecatetraensäure, Eicosatetraensäure, Docosapentaensäure, Eicosahexaensäure, Docosahexaensäure und ihren verschiedenen Estern sowie verschiedenen anderen Eicosanoiden, die gegebenenfalls in Form der Ester vorliegen, ausgewählt sind, beispielsweise PGE1 (Prostaglandin E1), PGA2 (Prostaglandin A2), Viprostol, 15-Monohydroxyeicosatetraensäure, 15-Monohydroxyeicosatriensäure, 15-Monohydroxyeicosapentaensäure und den Leukotrienen B5, C5 und D5.

21. Verfahren nach einem der vorhergehenden Ansprüche, dadurch gekennzeichnet, daß Cyclooxygenase-Inhibitoren eingesetzt werden, die unter den nichtsteroidalen entzündungshemmenden Mitteln ausgewählt sind.

22. Verfahren nach Anspruch 21, dadurch gekennzeichnet, daß die nichtsteroidalen entzündungshemmenden Mittel unter den Arylcarboxyderivaten, Pyrazolderivaten, Oxicamderivaten und Derivaten von Nicotinsäure ausgewählt sind.

23. Verfahren nach einem der vorhergehenden Ansprüche, dadurch gekennzeichnet, daß Stimulatoren oder Agonisten von Cyclooxygenasen verwendet werden, die unter den Metaboliten von Arachidonsäure, Stickstoffmonoxid und Stickstoffmonoxid-Donatoren, Stanozolol, Glutathion-Donatoren, Neuropeptiden und insbesondere VIP, Calcium-Ionophoren, Anthocyanosiden, Bioflavonoiden, FGA und plättchenaktiverenden Faktoren (PAF) ausgewählt sind.

24. Verfahren nach einem der vorhergehenden Ansprüche, dadurch gekennzeichnet, daß Lipoxygenase-Stimulatoren verwendet werden, die unter den Cytokinen, wie FGFβ, TGFβ und dem epidermalen Wachstumsfaktor (EGF), ausgewählt sind.

25. Verfahren nach einem der vorhergehenden Ansprüche, dadurch gekennzeichnet, daß als Substanz, die gleichzeitig als Lipoxygenase-Inhibitor und Cyclooxygenase-Stimulator wirkt, das 6-Chlor-2,3-dihydroxy-1,4-naphthochinon (CDNQ) verwendet wird.

26. Verfahren nach einem der Ansprüche 7 bis 25, dadurch gekennzeichnet, daß Substrate für Lipoxygenasen und Cyclooxygenasen verwendet werden, die unter den mehrfach ungesättigten Fettsäuren und insbesondere den Fettsäuren mit 20 Kohlenstoffatomen ausgewählt sind.

27. Verfahren nach Anspruch 26, dadurch gekennzeichnet, daß die mehrfach ungesättigten Fettsäuren unter Arachidonsäure, Dihomo-γ-linolensäure oder Eicosapentaensäure oder deren Gemischen ausgewählt sind.

28. Verfahren nach einem der Ansprüche 7 bis 27, dadurch gekennzeichnet, daß die Vorläuferverbindungen von Substraten für Lipoxygenasen und Cyclooxygenasen unter den essentiellen Fettsäuren und Phospholipiden der Zellmembran ausgewählt sind.

29. Verfahren nach Anspruch 28, dadurch gekennzeichnet, daß die essentiellen Fettsäuren unter Linolsäure, α-Linolensäure und β-Linolensäure oder deren Gemischen ausgewählt sind.

30. Verfahren nach Anspruch 28, dadurch gekennzeichnet, daß die Phospholipide unter Phosphatidylserin, Phosphatidylethanolamin, Phosphatidylcholin, Phosphatidylinosit und Diphosphatidylglycerin und deren Gemischen ausgewählt sind.

31. Zusammensetzungen, dadurch gekennzeichnet, daß sie mindestens einen Lipoxygenase-Inhibitor in Kombination mit mindestens einem Cyclooxygenase-Stimulator oder umgekehrt mindestens einen Cyclooxygenase-Inhibitor in Kombination mit mindestens einem Lipoxygenase-Stimulator enthalten.

32. Zusammensetzungen, dadurch gekennzeichnet, daß sie mindestens ein Substrat für Lipoxygenasen und Cyclooxygenasen und/oder eine Vorläuferverbindung eines Substrats für Lipoxygenasen und Cyclooxygenasen in Kombination mit mindestens einem Lipoxygenase-Inhbitor und/oder mindestens einem Cyclooxygenase-Stimulator oder umgekehrt mit mindestens einem Cyclooxygenase-Inhibitor und/oder mindestens einem Lipoxygenase-Stimulator enthalten.

33. Zusammensetzungen nach einem der Ansprüche 31 oder 32, dadurch gekennzeichnet, daß sie in einer für eine topische Anwendung geeigneten Form konfektioniert sind.

34. Zusammensetzungen nach einem der Ansprüche 31 bis 33, dadurch gekennzeichnet, daß es sich bei den Inhibitoren, Stimulatoren, Substraten und Vorläuferverbindungen von Substraten der Lipoxygenasen und Cyclooxygenasen um die Verbindungen nach einem der Ansprüche 12 bis 30 handelt.

35. Vorrichtungen oder "Kits" mit mehreren Abteilungen, dadurch gekennzeichnet, daß sie in einer ersten Abteilung mindestens einen Lipoxygenase-Inhibitor oder umgekehrt mindestens einen Cyclooxygenase-Inhibitor und in einer zweiten Abteilung mindestens einen Cyclooxygenase-Stimulator oder umgekehrt mindestens einen Lipoxygenase-Stimulator enthalten.

36. "Kits" nach Anspruch 35, dadurch gekennzeichnet, daß die Verbindungen der ersten und zweiten Abteilung in einer Form konfektioniert sind, die für eine topische Anwendung geeignet sind.

37. Vorrichtungen oder "Kits" mit mehreren Abteilungen, dadurch gekennzeichnet, daß sie in einer ersten Abteilung mindestens einen Lipoxygenase-Inhibitor und/oder mindestens einen Cyclooxygenase-Stimulator oder umgekehrt mindestens einen Cyclooxygenase-Inhibitor und/oder mindestens einen Lipoxygenase-Stimulator und in einer zweiten Abteilung mindestens ein Substrat für Lipoxygenasen und Cyclooxygenasen und/oder eine Vorläuferverbindung eines Substrats für Lipoxygenasen und Cyclooxygenasen enthalten.

38. "Kits" nach Anspruch 37, dadurch gekennzeichnet, daß die Verbindungen der ersten Abteilung in einer Form konfektioniert sind, die für eine topische Anwendung geeignet ist, und die Verbindungen der zweiten Abteilungen in einer Form konfektioniert sind, die für eine orale Verabreichung oder eine topische Anwendung geeignet ist.

39. "Kits" nach einem der Ansprüche 37 oder 38, dadurch gekennzeichnet, daß es sich bei den Inhibitoren, Stimulatoren, Substraten und Vorläuferverbindungen von Substraten der Lipoxygenasen und Cyclooxygenasen um die Verbindungen nach einem der Ansprüche 14 bis 32 handelt.

40. Nicht therapeutische Verwendung eines Lipoxygenase-Inhibitors mit Ausnahme von Ketoconazol oder Flavonoiden und/oder eines Cyclooxygenase-Stimulators als Mittel, um das Wachstum der Körperhaare und/oder Haare zu begünstigen und/oder den Haarausfall zu beschränken.

41. Nicht therapeutische Verwendung eines Lipoxygenase-Stimulators und eines Cyclooxygenase-Inhibitors als Mittel, um das Wachstum der Körperhaare und/oder Haare zu verlangsamen und/oder zu verhindern.

42. Verwendung nach einem der Ansprüche 40 oder 41, dadurch gekennzeichnet, daß die Inhibitoren oder Stimulatoren der Lipoxygenasen oder Cyclooxygenasen mit Hilfe von Zusammensetzungen oder "Kits" nach einem der Ansprüche 31 bis 39 eingesetzt werden.
